(19) Europäisches Patentamt
European Patent Office
Office européen des brevets

(11) Veröffentlichungsnummer: **0 067 234 B1**

(12) # EUROPÄISCHE PATENTSCHRIFT

(45) Veröffentlichungstag der Patentschrift: **17.04.85**

(51) Int. Cl.⁴: **C 12 Q 1/46**

(21) Anmeldenummer: **82900137.9**

(22) Anmeldetag: **23.12.81**

(86) Internationale Anmeldenummer: **PCT/JP 81/00405**

(87) Internationale Veröffentlichungsnummer: **WO 82/02212 (08.07.82 Gazette 82/17)**

(54) **VERFAHREN ZUR AKTIVITÄTSBESTIMMUNG VON CHOLINESTERASE.**

(30) Priorität: **25.12.80 JP 182910/80**

(43) Veröffentlichungstag der Anmeldung: **22.12.82 Patentblatt 82/51**

(45) Bekanntmachung des Hinweises auf die Patenterteilung: **17.04.85 Patentblatt 85/16**

(84) Benannte Vertragsstaaten: **CH DE FR GB LI NL SE**

(56) Entgegenhaltungen:
**DE - A - 2 527 993**
**JP - A - 54 136 895**
**US - A - 3 959 351**

**Agr. Biol. Chem, 33(5), P689 (1969)**
**Akabori Shiro hen "Koso Kenkyuho 4" Asakura Shoten P712-719**
**Chemical Abstracts, vol.71, no1, july 7, 1969, seite 289, Zusammenfassung Nr. 3153d COLUMBUS OHIO (US)**

(73) Patentinhaber: **SHINO-TEST LABORATORY CO., LTD., 52-1, Daita 6-chome Setagaya-ku, Tokyo 155 (JP)**

(72) Erfinder: **MOTONAGA, Hideo, 27-14, Oonodai 3-chome, Sagamiharashi Kanagawa 229 (JP)**
Erfinder: **NAITO, Masahiro, 1-6-46, Soubudai-Danchi 1-chome, Sagamiharashi Kanagawa 228 (JP)**

(74) Vertreter: **Boeters, Hans Dietrich, Dr. et al, Boeters, Bauer & Partner Thomas-Wimmer-Ring 14, D-8000 München 22 (DE)**

**EP 0 067 234 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Aktivitätsbestimmung von Cholinesterase (wird nachstehend als Ch-E abgekürzt) im Blutserum und ein Reagenz zur Ausführung des Verfahrens.

Cholinesterase ist ein Sammelbegriff für die Enzyme, welche Cholinester zu Cholin und organischer Säure hydrolysieren.

Im Körper ist echte Cholinesterase und Pseudo-cholinesterase vorhanden. Die echte Ch-E ist in den roten Blutkörperchen sowie im Nervensystem und im Muskel u. a. enthalten und weist eine spezifische Wirkung zur Zersetzung von Acetylcholin oder Acetyl-beta-methylcholin auf. Andererseits ist die Pseudo-Ch-E im Blutserum, in der Leber, im Pankreas u. a. enthalten und zersetzt spezifisch Benzoylcholin und hauptsächlich Cholinester von Fettsäuren mit einer längeren Kohlenstoffkette wie z. B. Butyrylcholin. Von den beiden Arten der Ch-E hat die quantitative Bestimmung der Aktivität von Pseudo-Ch-E besonders im Blutserum zur Untersuchung einer Leberschädigung eine erhebliche Bedeutung, weil die Aktivität der Pseudo-Ch-E bei Leberkrankheiten, besonders bei chronischer Schädigung des Leberparenchyms und Leberzirrhose, deutlich sinkt. Außerdem ist die Bestimmung bei Behandlung einer Vergiftung durch Verwendung einer Phosphor enthaltenden Verbindung in der Landwirtschaft und zur Untersuchung bei therapeutischer Behandlung mit einem Anti-cholinesterase-Präparat von Bedeutung.

Zur Aktivitätsbestimmung von Ch-E sind bereits Gasanalysen, delta-pH-Methoden, kolorimetrische Analysen, UV-Verfahren, Fluoreszenz-Verfahren und Elektrodenmethoden bekannt. Bei klinischen Routineuntersuchungen werden zunehmend die delta-pH-Methode und kolorimetrische Analyse angewendet. Die delta-pH-Methode, in welcher die durch Ch-E-Wirkung gebildete Essigsäure mit einem pH-Indikator bestimmt wird, benötigt eine komplizierte Korrektur der stark gekrümmten Eichkurve und weist eine etwas fragliche Treffsicherheit auf. Zur delta-pH-Methode vergleiche man z. B. Clinical Chemistry, Principles and Technics, 2. Auflage, Harper & Row, Hagerstown 1974, und zur Elektrodenmethode z. B. Clinica Chimica Acta 36 (1972) 405—408.

Als kolorimetrische Analyse gibt es das DTNB-Verfahren (Garrv. P.J. Clin. Chem. 11 (2): 91 (1965)) und das Enzym-Verfahren (Kunihide Gomi; Rinshobyori Sonderheft Bd. 29 145 (1977)).

Beim DNTB-Verfahren wird Acetylthiocholin, Butyrylthiocholin und Propionylthiocholin als Substrat verwendet und das gebildete Thiocholin wird mit 5,5'-Dithiobis-2-nitrobenzoesäure (DTNB) versetzt und die Intensität der Gelbfärbung im Vergleich mit Glutathion in einer reduzierten Form als Standard einer SH-Verbindung spektrometrisch gemessen. Die Richtigkeit der Bestimmung wird deswegen durch Bilirubin und eine reduzierende Substanz gestört und ferner ist die Beständigkeit der zu verwendenden Reagenzien kritisch.

Beim Enzym-Verfahren werden das Benzoylcholin und das ortho-Toluylcholin als Substrat verwendet, und das durch Enzymreaktion entstehende Cholin wird mit Cholinoxidase unter Entstehung von $H_2O_2$ zu Betain ungewandelt, wobei in Gegenwart der Peroxidase eine oxidierende Kondensation des 4-Aminoantipyrins mit Phenolen unter Verfärbung abläuft, welche spektrometrisch gemessen werden soll.

Die Bestimmung wird deswegen durch weiteres Cholin, welches durch Zersetzung des Phospholipids im Blutserum entsteht, beeinflußt, weil beim Verfahren Cholin mit Cholinoxidase umgesetzt werden soll. Außerdem wird sie auch durch eine reduzierende Substanz wie Bilirubin und Askorbinsäure beeinträchtigt. Das Verfahren ist ferner zur Bestimmung mit Hilfe eines Autoanalysators nicht geeignet.

Um die Nachteile der bisherigen Verfahren zu überwinden, wurde gemäß der japanischen Patentanmeldung 093 896/1980 ein Verfahren zur Bestimmung der Aktivität von Ch-E im Blutserum vorgeschlagen, in welchem p-Hydroxybenzoesäure, welche aus p-Hydroxybenzoylcholin als Substrat durch die enzymatische Wirkung von Ch-E gebildet wird, in Gegenwart eines Oxydationsmittels mit 4-Aminoantipyrin zu einer gefärbten Verbindung oxydierend kondensiert und der Färbungsgrad gemessen wird.

Gegenstand der Erfindung ist ein Verfahren zur enzymatischen Bestimmung von Cholinesterase, das dadurch gekennzeichnet ist, daß man die p-Hydroxybenzoesäure (p-HBA), welche aus p-Hydroxybenzoylcholin (p-HBC) als Substrat durch Einwirkung von Ch-E entsteht, in Gegenwart von Coenzym (Nicotinamidadenin-dinucleotid-phosphat in der reduzierten Form (NADPH)) mit der p-Hydroxybenzoesäure-Hydroxylase (p-HBH) oxydierend umsetzt, wobei die Verminderung des Extinktionswertes unter NADPH-Änderung zum Nicotinamid-adenin-dinucleotid-phosphat der oxydierten Form ($NADP^+$) oder der Sauerstoffverbrauch in der Reaktionsphase bestimmt wird.

Erfindungsgemäß zu verwendende p-Hydroxybenzoylcholinderivate (p-HBC) haben die allgemeine Formel

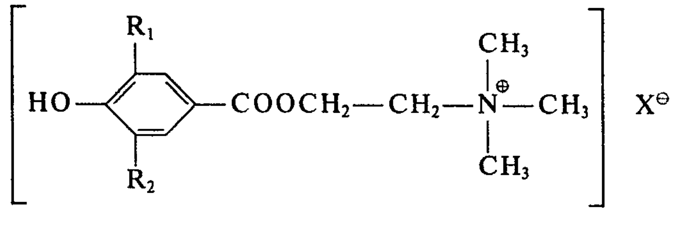

wobei $R_1$ und $R_2$ ein Wasserstoffatom und eines der beiden eine niedrige Alkoxy-Gruppe mit $C_1$ bis $C_4$ und X ein Halogenatom bedeuten.

Die Reaktionen bei der erfindungsgemäßen Bestimmung können mit dem folgenden Schema gezeigt werden.

$$(1) \quad \text{p-HBC} + H_2O \xrightarrow{(\text{Ch—E})} \text{p-HBA} + \text{Cholin}$$

$$(2) \quad \text{p-HBA} + O_2 + H^+ \xrightarrow{(\text{p-HBH})} \text{3,4-DBA} + H_2O$$

$$\text{NADPH} \curvearrowright \text{NADP}^+$$

Die nach der Reaktion (1) gebildete p-Hydroxybenzoesäure (p-HBA) wird in Gegenwart von Coenzym, NADPH, gemäß der Reaktion (2) durch die enzymatische Wirkung der p-Hydroxybenzoesäure-Hydroxylase zu 3,4-Dihydroxybenzoesäure (3,4-DBA) umgesetzt, wobei der Extinktionswert unter oxydierender Änderung von NADPH zu $NADP^+$ sinkt.

Um die Aktivität von Ch-E zu bestimmen, wird so die Extinktionsdifferenz bei 340 nm dadurch gemessen, daß nach Ablesung des ersten Extinktionswertes am Reaktionsanfang eine dauernde Ablesung des zweiten Extinktionswertes oder die zweite Ablesung erst nach einem bestimmten Zeitraum, gegebenenfalls unter Unterbrechung der Enzymreaktion durch Zusatz eines Reaktionshinderungsmittels, durchgeführt wird. Als Reaktionshinderungsmittel kommen in Frage:

Neostigmin, Eserin und organische Phosphor-Verbindungen, welche als hemmende Substanzen für die Ch-E-Aktivität bekannt sind.

Die Aktivität von Ch-E kann ferner durch Bestimmung der Geschwindigkeit des Verbrauches des im Reaktionsmedium gelösten Sauerstoffs durch Wirkung der p-Hydroxybenzoesäure-Hydroxylase mit Hilfe von Sauerstoffelektroden bestimmt werden.

Außerdem kann die Bestimmung der Verringerung der Fluoreszenz von NADPH mit einem photoelektrischen Fluorophotometer durchgeführt werden.

Das erfindungsgemäße Verfahren wird weder durch reduzierende Substanzen wie Bilirubin und Askorbinsäure im Blutserum noch durch Cholin aus Phospholipiden beeinträchtigt. Im übrigen könnte die Bestimmung der Ch-E-Aktivität mit einer kinetischen Bestimmungsmethode unter Verwendung eines chemischen Autoanalysators, mit welchem eine Reihe der Proben behandelt werden könnten, einfach und schnell durchgeführt werden.

Die erfindungsgemäß zu verwendende p-Hydroxybenzoesäure-Hydroxylase wird aus den Bakterien, welche zu Pseudomonas gehören, z. B. Ps. dacunhae, Ps. desmolytica, Ps. fluorescens, Ps. putida, hergestellt (Keiji Yano, Kei Arima, Agr. Biol. Chem., 33 (5): 689 (1969).

Die erfindungsgemäß zu verwendenden p-Hydroxybenzoylcholinderivate als Substrat weisen eine gute Wasserlösbarkeit und Lagerstabilität auf und besitzen eine Lagerfähigkeit von mehr als 2 Wochen in einer Wasserlösung an einer kühlen und dunklen Stelle. Die Herstellung der p-Hydroxybenzoylcholine wird nach den hierfür bekannten und üblichen Methoden erfolgen. Zum Beispiel kann man sie dadurch herstellen, daß man das p-Acetoxybenzoesäurechlorid mit dem N,N-Dimethyläthanolamin in einem organischen Lösungsmittel und dann unter Kühlung mit Methylhalogenid umsetzt und das Reaktionsgemisch in einem Gemisch von N,N-Dimethylformamid und Hydrazinhydrat löst.

Der optimale pH-Wert bei enzymatischer Reaktion der Ch-E mit dem erfindungsgemäß zu verwendenden Substrat ist zwischen 7,5 und 9,5, vorzugsweise 8,2. Als Puffer zur Beibehaltung des pH-Wertes der Reaktionslösung kommen z. B. in Frage:

Trishydroxymethylaminomethan, Glycylglycin, Phosphate, Borate, Pyrophosphorsäure, Salze der Barbitursäure, N,N-Bis-(2-hydroxyäthyl)-glycin, N-Tris-(hydroxymethyl)-methylglycin.

Ein Zusatz einer entsprechenden Menge von Flavin-adenindinucleotid (abgekürzt FAD) ist bei enzymatischer Bestimmung gemäß dem erfindungsgemäßen Verfahren vorteilhaft, weil die p-Hydroxybenzoesäure-Hydroxylase die mit FAD zusammenhängenden Enzyme darstellt.

Je nach dem anzuwendenden Enzym, welches jeweils durch Züchtung der oben genannten Bakterien hergestellt werden kann, ergibt erst ein Zusatz von Salicylsäure in einer Menge von etwa 5 bis 15 mM eine lineare Abhängigkeit der enzymatischen Reaktion.

Da die erfindungsgemäß zu verwendenden Reagenzien und Enzyme in einem weiten Konzentrationsbereich ohne Beeinträchtigung der Resultate der Aktivitätsbestimmung verwendet werden dürfen, ist eine strenge Begrenzung des Mengenbereiches nicht notwendig. Jedoch liegt vorzugsweise die Menge zwischen den nachfolgend angegebenen Bereichen:

| | |
|---|---|
| Für Enzyme | 200 bis 5000 U/l |
| vorzugsweise | 220 bis 700 U/l |
| Für Coenzyme | 0,1 bis 0,7 mM |
| vorzugsweise | 0,3 bis 0,5 mM |
| Für Substrat | 0,2 bis 20 mM |
| vorzugsweise | 0,5 bis 2 mM |
| Für FAD | 0,1 bis 30 µM |
| vorzugsweise | 1,0 bis 5 µM |

Fig. 1 der beigefügten Zeichnung zeigt die gegenseitige Abhängigkeit zwischen dem Reaktionszeitraum bei Inkubation unter Verwendung von 50 µl Blutserum und NADPH gemäß erfindungsgemäßem Verfahren.

Fig. 2 zeigt die gegenseitige Abhängigkeit zwischen dem erfindungsgemäßen Verfahren und dem vorbekannten Butyrylthiocholinverfahren.

Fig. 3 zeigt die Abnahme der Sauerstoffkonzentration der Lösung unter Verwendung des verdünnten Blutserums als Probe gemäß Beispiel 2.

Die nachfolgenden Beispiele erläutern die Erfindung weiter.

## Beispiel 1

### (1) Reagenzien

1. Zusammensetzung (Konzentration der gebrauchsfertigen Lösungen)

| | |
|---|---|
| Trishydroxymethylaminomethan-Maleinsäure-Pufferlösung | 50,0 mM |
| p-Hydroxybenzoylcholin-Jodid | 1,0 mM |
| FAD-2 Na | 0,003 mM |
| NADPH-4 Na | 0,3 mM |
| p-Hydroxybenzoesäure-Hydroxylase | 700,0 U/l |

2. Herstellung
   6,06 g Trishydroxymethylaminomethan werden in ungefähr 800 ml Wasser aufgelöst und mit einer wäßrigen Maleinsäurelösung auf pH 8,2 eingestellt. Dann werden 351 mg p-Hydroxybenzoylcholin-Jodid, 2,5 mg FAD-2 Na, 272 mg NADPH-4 Na und 700 U p-Hydroxybenzoesäure-Hydroxylase zugesetzt. Die so erhaltene Lösung wird mit Wasser auf 1000 ml aufgefüllt, um die Meßlösung herzustellen.

### (2) Arbeitsweise zur Bestimmung

3 ml der Meßlösung werden ungefähr 3 min auf 37° C vorgewärmt und mit 50 µl Blutserum gemischt. Sofort wird die Abnahme der Extinktion bei 340 nm unter Verwendung eines Autoanalysators verfolgt.

Wie die Fig. 1 zeigt, geht aus den Untersuchungsergebnissen mit den zwei Blutserumproben hervor, daß die Extinktion während des Reaktionsablaufs abnimmt. Nachdem die Reaktionsgeschwindigkeit konstant wird, soll die Aktivität der Ch-E aufgrund der folgenden Formel berechnet werden:

$$1\ \text{U/l} = \frac{\text{delta A 340} \times \text{Menge der Lösung} \times 1.000}{\text{molekularer Extinktionskoeffizient} \times \text{Menge des Blutserums}}\ .$$

In dieser Formel bedeutet delta A 340 eine Extinktions-Differenz im Zeitraum von 1 min bei 340 nm.

Der molekulare Extinktionskoeffizient von NADPH beträgt 6,22.

Nachstehend werden in der Tabelle 1 die Untersuchungsergebnisse einer Aktivitätsbestimmung mit Hilfe der oben erwähnten Arbeitsweise gezeigt, wobei jeweils 50 µl Serumprobe, welche mit $^1/_4$ bis $^4/_4$ Wasser verdünnt wurden, verwendet wurden.

Tabelle 1

| Serumverdünnung | $^1/_4$ | $^2/_4$ | $^3/_4$ | $^4/_4$ |
|---|---|---|---|---|
| Aktivität der Ch-E (U/l) | 142 | 294 | 412 | 569 |

In Fig. 2 werden die Untersuchungsergebnisse der Aktivitäten der Ch-E von 15 Serumproben im Zusammenhang mit der Butyrylthiocholin DTNB-Methode (G. Szasz: Clin. Chem. Acta., 19 : 191 : 1968) gezeigt.

Beispiel 2

Die Untersuchung des Beispiels 1 wurde unter Abänderung des pH-Wertes der Trishydroxymethylaminomethan-Maleinsäure-Pufferlösung auf 8,5 und unter Verwendung von 25 mg FAD-2 Na und 220 U p-Hydroxybenzoesäure-Hydroxylase wiederholt.

Die Ergebnisse waren gut vergleichbar mit denen im Beispiel 1.

Beispiel 3

(1) Reagenzien

1. Zusammensetzung (Konzentration der gebrauchsfertigen Lösungen)

| | |
|---|---|
| Trishydroxymethylaminomethan-Maleinsäure-Pufferlösung | 50,0 mM |
| p-Hydroxybenzoylcholin-Jodid | 1,0 mM |
| FAD-2 Na | 0,003 mM |
| NADPH-4 Na | 1,5 mM |
| p-Hydroxybenzoesäure-Hydroxylase | 700,0 U/l |

2. Herstellung
6,06 g Trishydroxymethylaminomethan werden in ungefähr 800 ml Wasser aufgelöst und auf pH 8,2 mit einer wäßrigen Maleinsäurelösung eingestellt. Dann werden 351 mg p-Hydroxybenzoylcholin-Jodid, 2,5 mg FAD-2 Na, 272 mg NADPH-4 Na und 700 U p-Hydroxybenzoesäure-Hydroxylase zugesetzt. Die so erhaltene Lösung wird mit Wasser auf 1000 ml aufgefüllt, um die Meßlösung herzustellen.

(2) Arbeitsweise zur Bestimmung

Die Meßlösung wird auf 37° C erwärmt und in einer Kammer, in welche der Sauerstoffdetektor ragt, eingeführt, 20 µl Serum werden zugefügt. Die Abnahme der Sauerstoffkonzentration der Lösung wird mit dem Sauerstoffdetektor (Stadt. Glucoseanalysator S-80: Hersteller: AIC-Werke) gemessen.

Wie in Fig. 3 der Zeichnung gezeigt, nimmt die Sauerstoffkonzentration (Volt.) im Verlauf der Reaktion ab.

Nachstehend werden in der Fig. 3 und Tabelle 2 die Untersuchungsergebnisse einer Aktivitätsbestimmung mit Hilfe der im Beispiel 1 erwähnten Arbeitsweise gezeigt, wobei jeweils Serumproben, welche mit $^1/_4$ bis $^4/_4$ Wasser verdünnt wurden, verwendet wurden. Die Ergebnisse waren ganz befriedigend.

(3) Umrechnung der Aktivitäts-Einheiten von Ch-E

1. Die oben genannte Arbeitsweise zur Bestimmung wird mit 20 µl Serum mit einer vorbekannten Einheit durchgeführt. Die Aktivität der Probe wird im Verhältnis zum gemessenen Wert gezeigt.

$$\text{Serumprobe U/l} \quad a \times \frac{c}{b},$$

Standardserum = a U/l,
Meßwert des Standardserums = b mV/min,
Meßwert der Serumprobe = c mV/min.

2. Die Aktivität der Serumprobe wird ferner aufgrund der Abnahme der Sauerstoffkonzentration der Lösung während der Reaktion von 20 µl p-Hydroxybenzoesäure mit einer bekannten Konzentration umgerechnet.

$$\text{Serumprobe U/l} = \frac{a \times c \times 10^3}{b} \left(\frac{c}{b} \times 0{,}02\,a \times 5 \times 10^4\right),$$

wobei

| | |
|---|---|
| p-Hydroxybenzoesäure | = a mM |
| Abnahme der Sauerstoffkonzentration im Verlauf der Reaktion | = b mV |
| Meßwert der Serumprobe | = c mV/min |

Tabelle 2

| Serumverdünnung | 1/4 | 2/4 | 3/4 | 4/4 |
|---|---|---|---|---|
| Aktivität der Ch-E (U/l) | 3,0 | 3,9 | 5,0 | 7,0 |

Beispiel 4

Das Beispiel 3 wurde wiederholt, jedoch in Abänderung des pH-Wertes auf 8,5 und unter Verwendung von 25 mg FAD-2 N und 220 U der p-Hydroxybenzoesäure-Hydroxylase. Die Untersuchungsergebnisse waren vergleichbar mit denen im Beispiel 3.

Beispiel 5

(1) Reagenzien

1. Zusammensetzung (Konzentration der gebrauchsfertigen Lösungen)

| | | |
|---|---|---|
| i) | Trishydroxymethylaminomethan-Maleinsäure-Pufferlösung | 50,0 mM |
| | FAD-2 Na | 0,003 mM |
| | p-Hydroxybenzoesäure-Hydroxylase | 1.000 U/l |
| | NADPH-4 Na | 0,45 mM |
| ii) | Trishydroxymethylaminomethan-Maleinsäure-Pufferlösung | 50,0 mM |
| | p-Hydroxybenzoylcholin-Jodid | 1,5 mM |
| iii) | Wäßrige Lösung von Neostigmin | 20,0 mM |

2. Herstellung

i) 6,06 g Trishydroxymethylaminomethan werden in ungefähr 800 ml Wasser gelöst und mit einer wäßrigen Lösung von Maleinsäure auf pH 8,2 eingestellt.
Dann werden 2,5 mg FAD-2 Na, 272 mg NADPH-4 Na und 1.000 U p-Hydroxybenzoesäure-Hydroxylase zugesetzt. Die so erhaltene Lösung wird mit Wasser auf 1.000 ml aufgefüllt, um die Meßlösung herzustellen.

ii) 6,06 g Trishydroxymethylaminomethan werden in ungefähr 800 ml Wasser aufgelöst und mit einer wäßrigen Maleinsäurelösung auf pH 8,2 eingestellt. Dann werden 526,5 mg p-Hydroxybenzoylcholin-Jodid zugesetzt. Die so erhaltene Lösung wird mit Wasser auf 1.000 ml aufgefüllt.

iii) 6 g Neostigmin werden in ungefähr 1.000 ml Wasser aufgelöst.

(2) Arbeitsweise zur Bestimmung

i) Reagenzien-Leerwert ($A_B$)
1,0 ml Reagenzien i) werden mit 20 µl Wasser gemischt; nach 5 min werden bei 37°C 0,5 ml der Reagenzien ii) zugesetzt. Nach Inkubierung von 10 min bei 37°C werden 0,1 ml Reagenzien iii) zugesetzt, und die Reaktion wird sofort zum Stillstand gebracht. Dann wird die Extinktion ($A_B$) bei 340 nm gemessen.

ii) Probe
1,0 ml Reagenzien i) werden jeweils in 20 µl Serumprobe und 20 µl Standardserum gemischt, und dann werden nach 5 min bei 37°C 0,5 ml Reagenzien ii) zugesetzt. Nach Inkubierung von 10 min bei 37°C werden 0,1 ml Reagenzien iii) zugesetzt, und die Reaktion wird zum Stillstand gebracht. Die Extinktionen der Serumprobe ($A_I$) und des Standardserums ($A_S$) werden sofort bei 340 nm gemessen.

(3) Berechnung der Aktivität der Ch-E

Die Aktivität der Ch-E im Serum wird nach der folgenden Formel berechnet.

$$\frac{A_B - A_I}{A_B - A_S} \times \text{Einheit des Standardserums.}$$

Nachstehend werden in der Tabelle 3 die Untersuchungsergebnisse einer Aktivitätsbestimmung mit Hilfe der oben genannten Arbeitsweise gezeigt, wobei jeweils 50 μl Serumprobe, welche mit Wasser einer Menge von $^1/_4$ bis $^4/_4$ verdünnt wurde, verwendet wurden.

Tabelle 3

| | | | | |
|---|---|---|---|---|
| Serumverdünnung | $^1/_4$ | $^2/_4$ | $^3/_4$ | $^4/_4$ |
| Aktivität der Ch-E (U/l) | 155 | 314 | 461 | 617 |

Beispiel 6

Die gleiche Untersuchung der Extinktion gemäß Beispiel 5 wurde wiederholt, jedoch in Abänderung des pH-Wertes auf 8,5 und unter Verwendung von 0,03 mM FAD-2 Na und 330 U p-Hydroxybenzoesäure-Hydroxylase. Die Ergebnisse waren wie im Beispiel 5.

**Patentansprüche**

1. Verfahren zur Aktivitätsbestimmung von Cholinesterase unter Verwendung von p-Hydroxybenzoylcholinderivaten der allgemeinen Formel

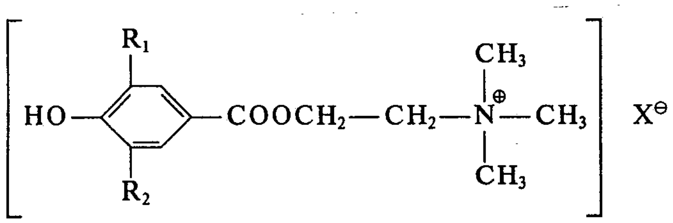

als Substrat,
wobei $R_1$ und $R_2$ ein Wasserstoffatom oder eines der beiden eine niedrige Alkoxygruppe mit 1 bis 4 Kohlenstoffatomen und X ein Halogenatom bedeuten, dadurch gekennzeichnet, daß man die p-Hydroxybenzoesäure, welche aus dem Substrat unter Einwirkung von Cholinesterase gebildet wird, mit p-Hydroxybenzoesäure-Hydroxylase in Gegenwart der reduzierten Form des Coenzyms Nikotinamidadenin-dinucleotidphosphat oxidierend umsetzt, wobei

(a) die Extinktionsabnahmedifferenz,
(b) die Fluoreszenzabnahme,
(c) die Reaktionskinetik (Autoanalyse) oder
(d) der Sauerstoffverbrauch in der Reaktionslösung bei der Umwandlung des Nicotinamid-adenin-dinucleotidphosphates von der reduzierten Form abgelesen oder gemessen werden.

2. Verfahren gemäß Anspruch 1, dadurch gekennzeichnet, daß die Extinktion bei 340 nm gemessen wird.
3. Verfahren gemäß Anspruch 1, dadurch gekennzeichnet, daß die Geschwindigkeit des Sauerstoffverbrauches in der Reaktionslösung mit Hilfe einer Sauerstoffelektrode gemessen wird.
4. Verfahren gemäß Anspruch 1, dadurch gekennzeichnet, daß die Reaktion der p-Hydroxybenzoesäure-Hydroxylase in Gegenwart von FAD durchgeführt wird.
5. Verfahren gemäß Anspruch 1, dadurch gekennzeichnet, daß die Reaktion der Cholinesterase mit Substrat bei einem pH-Wert von 7,5 bis 9,5, insbesondere von 8,2 in Gegenwart von Puffern durchgeführt wird.
6. Reagenz zur Bestimmung der Cholinesterase gemäß Anspruch 1, dadurch gekennzeichnet, daß es aus p-Hydroxybenzoylcholin, p-Hydroxybenzoesäure-Hydroxylase, Coenzym Nicotinamid-adenin-dinucleotidphosphat in einer reduzierten Form und Puffer besteht.

7. Reagenz nach Anspruch 6, dadurch gekennzeichnet, daß FAD und/oder Salicylsäure zusätzlich enthalten ist.

8. Reagenz nach Anspruch 6, dadurch gekennzeichnet, daß es 0,02 bis 20 mM p-Hydroxybenzoylcholin, 0,1 bis 0,7 mM Nicotinamid-adenin-dinucleotidphosphat in einer reduzierten Form, 200 bis 5000 U/l p-Hydroxybenzoesäure-Hydroxylase und 0,1 bis 30 μM FAD enthält.

**Claims**

1. A method of determining the activity of choline esterase, using p-hydroxybenzoylcholine derivatives having the following general formula:

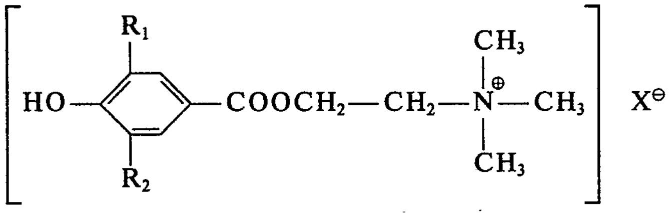

as a substrate,
wherein $R_1$ and $R_2$ denote a hydrogen atom or one of the two denotes a low alkoxy group having from 1 to 4 carbon atoms and X denotes a halogen atom, characterised in that the p-hydroxybenzoic acid which is formed from the substrate, under the action of choline esterase, is subjected to oxidising reaction with p-hydroxybenzoic acid hydroxylase in the presence of the reduced form of the coenzyme nicotinamide adenine dinucleotide phosphate, wherein

(a) the extinction reduction difference,
(b) the fluorescence reduction,
(c) the reaction kinetics (auto-analysis) or
(d) the oxygen consumption in the reaction solution upon conversion of the nicotinamide adenine dinucleotide phosphate from the reduced form is read off or measured.

2. A method according to claim 1 characterised in that extinction is measured at 340 nm.

3. A method according to claim 1 characterised in that the rate of oxygen consumption in the reaction solution is measured by means of an oxygen electrode.

4. A method according to claim 1 characterised in that the reaction to the p-hydroxybenzoic acid hydroxylase is performed in the presence of FAD.

5. A method according to claim 1 characterised in that the reaction of the choline with the substrate is performed at a pH-value of 7.5 to 9.5, in particular 8.2, in the presence of buffers.

6. Reagent for evaluating the choline esterase according to claim 1 characterised in that it comprises p-hydroxybenzoyl choline, p-hydroxybenzoic acid hydroxylase, the coenzyme nicotinamide adenine dinucleotide phosphate in a reduced form and buffer.

7. Reagent according to claim 6 characterised in that FAD and/or salicylic acid is additionally included.

8. Reagent according to claim 6 characterised in that it contains from 0.02 to 20 mM of p-hydroxybenzoyl choline, from 0.1 to 0.7 mM of nicotinamide adenine dinucleotide phosphate in a reduced form, from 200 to 5000 U/l of p-hydroxybenzoic acid hydroxylase and from 0.1 to 30 μM of FAD.

**Revendications**

1. Procédé de détermination de l'activité de cholinestérase en utilisant des dérivés de p-hydroxybenzoylcholine de formule générale:

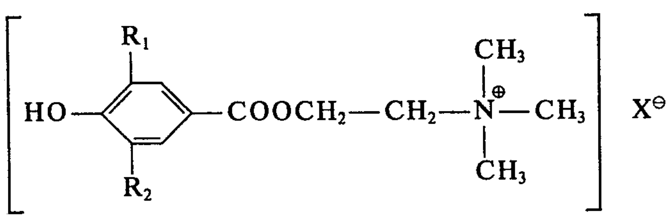

comme substrat,

dans laquelle $R_1$ et $R_2$ désignent un atome d'hydrogène ou un des deux un groupe alcoxy-inférieur avec 1 à 4 atomes de carbone et X un atome d'halogène, caractérisé en ce qu'on convertit par oxydation l'acide p-hydroxybenzoïque, qui est formé à partir du substrat sous l'action de la cholinestérase, avec de l'hydroxylase d'acide p-hydroxybenzoïque en présence de la l'hydroxylase d'acide p-hydroxybenzoïque en présence de la forme réduite du co-enzyme constitué par le phosphate de nicotinamide-adénine-dinucléotide, en lisant ou en mesurant:

a) la différence de réduction d'extinction,
b) la réduction de fluorescence,
c) la cinétique de la réaction (auto-analyse), ou
d) la consommation d'oxygène dans la solution réactionnelle pendant la transformation du phosphate de nicotinamide-adéninedinucléotide de forme réduite.

2. Procédé selon la revendication 1, caractérisé en ce que l'extinction est mesurée à 340 nm.

3. Procédé selon la revendication 1, caractérisé en ce que la vitesse de consommation d'oxygène dans la solution réactionnelle est mesurée à l'aide d'une électrode à oxygène.

4. Procédé selon la revendication 1, caractérisé en ce que la réaction de l'hydroxylase d'acide p-hydroxybenzoïque est effectuée en présence de FAD.

5. Procédé selon la revendication 1, caractérisé en ce que la réaction de la cholinestérase avec le substrat est effectuée en présence d'un tampon avec une valeur du pH de 7,5 à 9,5, en particulier de 8,2.

6. Réactif pour la détermination de la cholinestérase selon la revendication 1, caractérisé en ce qu'il se compose de p-hydroxybenzoylcholine, d'hydroxylase d'acide p-hydroxybenzoïque, d'un co-enzyme constitué par le phosphate de nicotinamide-adénine-dinucléotide sous forme réduite et d'un tampon.

7. Réactif selon la revendication 6, caractérisé en ce qu'il contient additionnellement FAD et/ou de l'acide salicylique.

8. Réactif selon la revendication 6, caractérisé en ce qu'il contient de 0,02 à 20 mM de p-hydroxybenzoylcholine, de 0,1 à 0,7 mM de phosphate de nicotinamide-adénine-dinucléotide sous forme réduite, de 200 à 5000 U/l d'hydroxylase d'acide p-hydroxybenzoïque et de 0,1 à 30 μM de FAD.

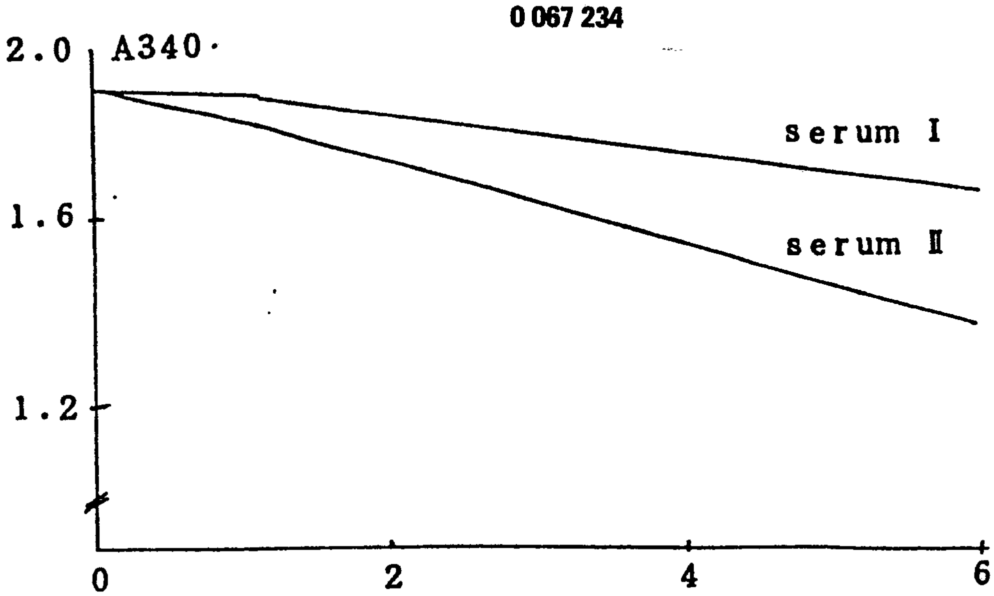


Fig.1

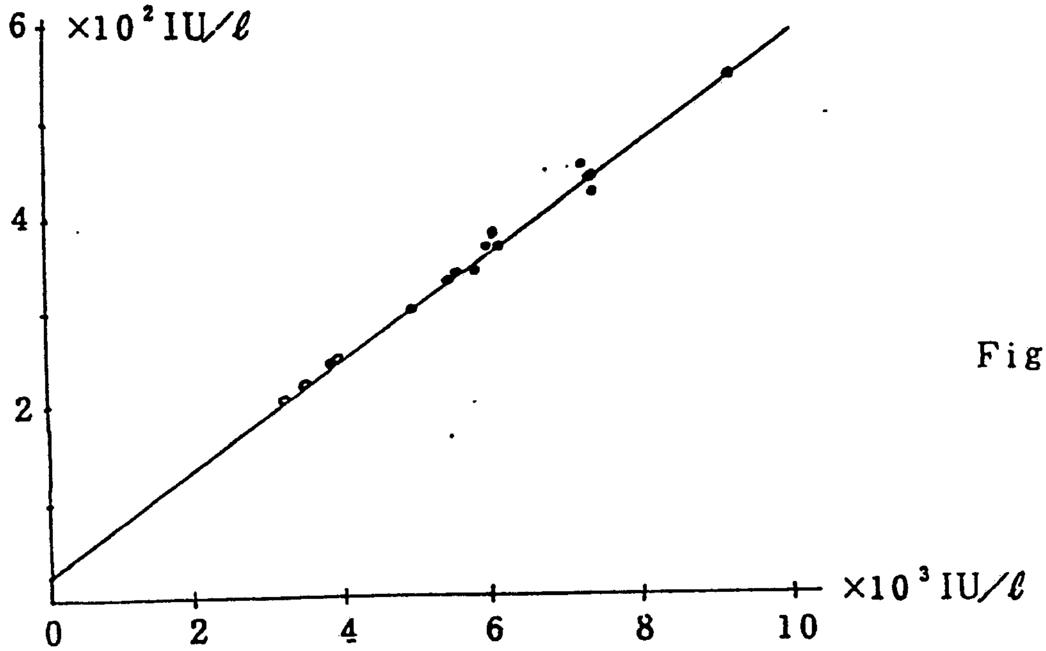


Fig.2

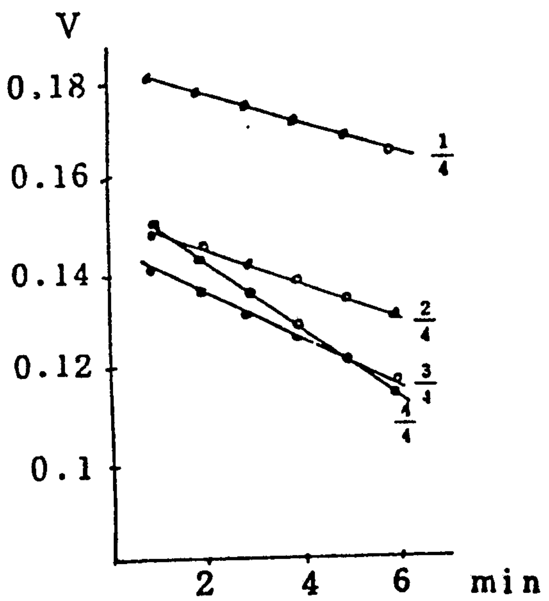


Fig.3